# EUROPEAN PATENT SPECIFICATION

(11) **EP 3 493 801 B1**
(45) Date of publication and mention of the grant of the patent: **31.12.2025**
(21) Application number: 17837761.0
(22) Date of filing: 04.08.2017
(51) Int. Cl.: A61K 31/4196, A61K 31/4412, A61K 31/047, A61K 31/05, A61K 31/075, A61K 31/132, A61K 31/16, A61K 31/192, A61K 31/357, A61K 31/426, A61P 39/04

(54) **DOSAGE REGIMENS WITH (S)-4,5-DIHYDRO-2-[2-HYDROXY-4-(3,6-DIOXAHEPTYLOXY)PHENYL]-4-METHYL-4-THIAZOLECARBOXYLIC ACID FOR USE IN TREATING IRON OVERLOAD**
DOSIERUNGSSCHEMA MIT (S)-4,5-DIHYDRO-2-[2-HYDROXY-4-(3,6-DIOXAHEPTYLOXY)PHENYL]-4-METHYL-4-THIAZOLCARBONSÄURE ZUR VERWENDUNG IN DER BEHANDLUNG VON EISENÜBERLADUNG
RÉGIMES POSOLOGIQUES AVEC DE L'ACIDE (S)-4,5-DIHYDRO-2-[2-HYDROXY-4-(3,6-DIOXAHEPTYLOXY)PHENYL]-4-METHYL-4-THIAZOLECARBOXYLIQUE POUR L'UTILISATION DANS LE TRAITEMENT D'UNE SURCHARGE EN FER

(30) Priority: 05.08.2016 US 201662371274 P; 05.08.2016 US 201662371280 P
(43) Date of publication of application: 12.06.2019
(73) Proprietor: Abfero Pharmaceuticals, Inc., Lexington, Massachusetts 02421 (US); University of Florida Research Foundation, Inc., Gainesville, FL 32611 (US)
(72) Inventor: BURKE, Steven Keith, Lexington, Massachusetts 02421 (US); BERGERON, Raymond J., Jr., Gainesville, Florida 32611 (US)
(74) Representative: Mewburn Ellis LLP
(86) International application number: PCT/US2017/045495
(87) International publication number: WO 2018/027132

(56) References cited:
- WO-A1-02/02114
- US-A1- 2005 175 684
- US-A1- 2010 316 700
- US-A1- 2012 184 586
- US-A1- 2015 259 306
- BERGERON R J ET AL: "HBED: The continuing development of a potential alternative to deferoxamine for iron-chelating therapy", BLOOD,, vol. 93, no. 1, 1 January 1999 (1999-01-01), pages 370 - 375, XP009518853, ISSN: 0006-4971
- WHITESIDE DOUGLAS P ET AL: "Clinical evaluation of the oral iron chelator deferiprone for the potential treatment of iron overload in bird species", JOURNAL OF ZOO AND WILDLIFE MEDICINE, THE ASSOCIATION, LAWRENCE,KS, US, vol. 35, no. 1, 29 February 2004 (2004-02-29), pages 40 - 49, XP009518842, ISSN: 1042-7260, DOI: 10.1638/02-031
- CONNELLY JOHN ET AL: "Comparative benefits of the non-human primate in long-term toxicity studies with iron chelators: 12-month studies with deferiprone", BLOOD,, vol. 104, no. 11, Part 2, 16 November 2004 (2004-11-16), pages 33B, XP009518844, ISSN: 0006-4971, DOI: 10.1182/BLOOD.V104.11.3785.3785
- AGUILAR-DE-LEYVA ANGELA ET AL: "A new deferiprone controlled release system obtained by ultrasound-assisted compression", PHARMACEUTICAL DEVELOPMENT AND TECHNOLOGY, INFORMA HEALTHCARE, US, vol. 19, no. 6, 31 August 2014 (2014-08-31), pages 728 - 734, XP009518840, ISSN: 1097-9867, DOI: 10.3109/10837450.2013.829091
- RAYMOND J. BERGERON ET AL: "Substituent Effects on Desferrithiocin and Desferrithiocin Analogue Iron-Clearing and Toxicity Profiles", JOURNAL OF MEDICINAL CHEMISTRY, vol. 55, no. 16, 23 August 2012 (2012-08-23), US, pages 7090 - 7103, XP055345573, ISSN: 0022-2623, DOI: 10.1021/jm300509y
- BARTON: "Erratum Drug evaluation: Deferitrin (GT-56-252; NaHBED) for iron overload disorders", IDRUGS, vol. 10, no. 7, 2007, pages 480 - 490, XP009183135, Retrieved from the Internet <URL:https://pdfs.semanticscholar.org/b471/6115f3399ae029ae416f7843fcc19f4c58ee.pdf> [retrieved on 20171002]
- REINOFF JR ET AL.: "A phase 1 dose-escalation study: safety, tolerability, and pharmacokinetics of FBS0701, a novel oral iron chelator for the treatment of transfusional iron overload", HAEMATOLOGICA, vol. 96, no. 4, April 2011 (2011-04-01), pages 521 - 525, XP055203044, Retrieved from the Internet <URL:https://www.ncbi.nlm.nih.gov/pmc/articles/PMC3069228/pdf/0960521.pd> [retrieved on 20171001]

## Description

### 1. RELATED APPLICATIONS

This application claims the priority benefit of U.S. provisional application nos. 62/371,274, filed August 5, 2016, and 62/371,280, filed August 5, 2016.

### 2. BACKGROUND

Iron and other metals are essential nutrients for health. Metals form cofactors for enzymes known as metaloenzymes and are essential constituents of other metal containing proteins such as the cytochromes, hemoglobin, and myoglobin. Many of these metal containing proteins are critical for normal cellular function. Metal deficiencies, usually due to insufficient gastrointestinal absorption (e.g., calcium in celiac sprue) or loss due to conditions affecting the gastrointestinal, kidney, or skin (e.g., magnesium in diarrheal conditions or kidney with diuretic use) have well-defined clinical syndromes with characteristic signs and symptoms. Metal accumulation disorders also occur where excessive amounts of metals accumulate within the tissues of the body usually secondary to excessive absorption (*e.g.,* iron in hereditary hemochromatosis) or impaired secretory mechanisms (*e.g.,* copper in Wilson's disease) likewise causing well-defined clinical syndromes with characteristic signs and symptoms.

Iron is a good example for other metals. Iron, although abundant in nature, is difficult to absorb from the diet. Poor iron absorption from the diet or loss due to bleeding (*e.g.,* menstruation) can lead to deficiency. Approximately 11% of persons in the US have iron deficiency and 1-2% deficiency severe enough to cause anemia. Iron overload is much less common and occurs most often due to abnormally up-regulated iron absorption as in hereditary hemochromatosis or secondary to anemia due to abnormal red blood cell (RBC) production. Anemia results in down-regulated hepatic hepcidin, a key-regulator of iron absorption. Decreased blood hepcidin results in increased intestinal cell ferroportin (iron transporter) expression resulting in excessive iron absorption by enterocytes of the intestine. The iron overload is severely exacerbated in the subset of patients who require blood transfusion as a treatment for the anemia. The average unit of packed RBCs contains 200-250 mg of iron. To put this in perspective, an average person's body contains 3 grams of iron. As there is no excretory mechanism for iron, iron overload rapidly ensues with RBC transfusions. These disorders of RBC production include thalassemias, sickle-cell disease, Diamond-Blackfan anemia, congenital sideroblastic anemias, congenital dyserythropoietic anemias, myelodysplastic syndrome (MDS), and aplastic anemias.

Iron is normally transported in the body bound to the protein transferrin. Transferrin receptors on cells bind to and internalize the transferrin-iron complex, liberate the iron intracellularly, and release the transferrin. The percentage of circulating transferrin that is complexed with iron, the transferrin saturation, is used clinically as an indicator of body iron status. Normal transferrin saturation ranges from approximately 20 to 50%. Values below this suggest iron deficiency and those above suggest iron excess or overload. Inside cells, iron is stored in protein encased spheres known as ferritin. Ferritin can leak from cells and is used as an indicator of body iron status. The normal range is 20 to 200 ng/mL and, as with transferrin saturation, values below and above this range suggest iron insufficiency or excess. Iron is normally always bound to transferrin or stored in ferritin. Iron that is not bound in the circulation to transferrin, non-transferrin bound iron (NTBI), is bound to low molecular weight substance such as citrate. Some NTBI is capable of catalyzing (*i.e.,* producing) reactive oxygen species and is referred to as "catalytic iron." The reactive oxygen species sometime referred to as "free radicals," damage lipids, proteins, deoxyribonucleic acid (DNA), and subcellular organelles leading to cellular injury, apoptosis, necrosis, and inflammation. Signs and symptoms of iron overload include fatigue, joint pain, impotence, osteoporosis, diabetes, heart failure, and cirrhosis of the liver.

Prior to the advent of effective agents to chelate iron and clear it from the body, patients with β-thalassemia, a severe form of transfusion related iron-overload, succumbed to heart failure as early as the second decade of life. Subsequent to the introduction of iron chelators in the early 1970s, there has been a progressive increase in longevity. Still, data from the United Kingdom (UK) Thalassaemia Register reported that approximately 50% of patients with β-thalassemia will die before age 35 years, predominantly of cardiac disease. The key predictor of survival is the body iron burden. In 1 study, patients who maintained serum ferritin (a marker of body iron content) below 2500 ng/mL had a cardiac disease-free survival of 91% at 15 years versus 20% in those with ferritin exceeding 2500 ng/mL.

Iron chelators are small molecules that form complexes with iron and promote iron excretion. They are capable of binding NTBI in the circulation and within cells. Before initiation of treatment, the iron load in the body is determined by measuring liver iron content (LIC) and cardiac iron content. LIC is measured by magnetic resonance imaging or MRI. Cardiac iron is also determined by MRI and reported as a T2* score. A lower score indicates greater cardiac iron content. The goal of iron chelation is dependent on the severity of iron overload. In severely iron overloaded patients (LIC > 7 or T2*<10), the goal is to create a state of negative body iron balance. In patients with acceptable iron load (LIC 3-7 and T2*>10), the goal is to create a balance between iron input (from diet and blood transfusions) and iron output from the chelator.

Three iron chelator compounds are approved for use in the US and other countries - deferoxamine (*e.g.,* Desferal^{®}), deferiprone (*e.g.,* Ferriprox^{®}), and deferasirox (*e.g.,* Exjade^{®}, Jadenu^{®}). All 3 chelators are effective to varying degrees, but are associated with significant toxic side effects that are dose-related and also related to iron status. Excessive iron chelation that leads to LIC < 3 has been shown to be extremely dangerous and in some cases fatal.

Deferoxamine, the first approved iron chelator, was a major breakthrough in the treatment of iron overload and extended life expectancy substantially. Deferoxamine is indicated in the US for the treatment of acute iron intoxication and of chronic iron overload due to transfusion-dependent anemias. Unfortunately, deferoxamine has poor oral bioavailability and a short half-life requiring continuous parenteral administration (intravenously [IV] or subcutaneously [SC]) for 8-10 hours per day 5 to 7 days per week. Side effects include local inflammatory and allergic reactions, systemic allergic reactions, ocular and auditory disturbances, increase in serum creatinine, renal failure, renal tubular disorders, growth retardation, and respiratory distress. Noncompliance is a significant problem, resulting in suboptimal efficacy. As many as half of patients on deferoxamine may have levels of iron overload associated with premature cardiac death. Survival is closely related to compliance. In 1 study, survival at age 30 years was 60% in compliant patients but only 10% in non-compliant patients.

The burdensome nature of deferoxamine treatment and the strong association of survival with compliance motivated the search for alternative treatments. Deferiprone was the first approved orally-active iron chelator and is indicated for the treatment of patients with thalassemia syndrome when current chelation therapy is inadequate. Deferiprone has low iron chelator efficiency and a short elimination half-life (t_{1/2}=1.9 hours) and therefore must be administered at a dose of 75 to 99 mg/kg/day divided into 3 daily doses. Side effects include nausea, vomiting and abdominal pain, increased alanine aminotransferase (ALT), arthralgia, neutropenia, and agranulocytosis requiring frequent monitoring to avoid potentially fatal infections. Furthermore, deferiprone is pregnancy category D secondary to embryofetal deaths and malformations in nonclinical studies. Of the 3 available chelators, deferiprone may be the most effective at chelating cardiac iron, but is least effective at clearing liver iron. Perhaps as a result of this safety and efficacy profile, deferiprone is the least used iron chelator and is mainly used as a supplement to deferoxamine.

Deferasirox is a more recently developed oral iron chelator and has a potency and pharmacokinetic profile that allows once daily dosing. Deferasirox was shown to be non-inferior to deferoxamine in the subset of patients with elevated LIC. The approval of deferasirox represented a major advance in the treatment of iron overload, and it is currently indicated for the treatment of chronic iron overload due to blood transfusion in patients 2 years of age and older. The prescribing information includes a warning that deferasirox may cause renal and hepatic impairment, including organ failure and gastrointestinal hemorrhage. In some cases, these reactions are fatal. As a result, close monitoring with frequent tests of renal and hepatic function are required during treatment. Deferasirox is contraindicated for patients with creatinine clearance <40 mL/min or serum creatinine >2 times the upper limit of normal, poor performance status high-risk MDS, advanced malignancy, or very low platelet counts. Approximately one-third of patients will experience a persistent increase in serum creatinine of 33% or greater which may require dose reduction, interruption, or discontinuation. Abdominal pain, diarrhea, nausea, vomiting, and rash are frequent side effects that may result in noncompliance or under-dosing. Deferasirox is available as wafer-like tablets that must be completely dispersed in water, orange juice, or apple juice and consumed 30 minutes prior to a meal to avoid food effects on bioavailability. Recently, Jadenu tablets were approved which allows oral dosing of deferasirox dose without the need to disperse the dosage form. Since the Jadenu and Exjade contain the same active ingredient the side effect profiles are similar. Despite these limitations, deferasirox has become the most widely used iron chelator in the US.

Given the limitations of the 3 approved iron chelators, there is an important unmet medical need for safe and effective iron chelators. An ideal iron chelator would have high iron chelating efficiency with oral dosing, high penetration into all organs adversely affected by iron overload (*e.g.,* liver, heart, pancreas, and kidney), and minimal toxicity, particularly to the renal, hepatic, and gastrointestinal systems. This has not proven easy to accomplish as evidenced by previous failure of products advanced into clinical development. HBED (N,N8-bis(2-hydroxybenzyl)ethylenediamine-N,N8-diacetic acid) development was discontinued after phase 1 due to lack of sufficient oral bioavailability. Deferitrin (4,5-dihydro-2-(2,4-dihydroxyphenyl)-4-methylthiazole-4(S)-carboxylic acid), an iron chelator with good oral bioavailability, was discontinued after a phase 2 study demonstrated kidney injury. SPD602 ((S)-2-(2-hydroxy-3-(2-(2-(2-methoxyethoxy)ethoxy)ethoxy)phenyl)-4-methyl-4,5-dihydrothiazole-4-carboxylic acid), formerly FBS0701, completed a phase 2 study but failed to significantly reduce LIC in patients with β-thalassemia. SP-420 ((S)-4,5-dihydro-2-[2-hydroxy-4-(3,6-dioxaheptyloxy)phenyl]-4-methyl-4-thiazolecarboxylic acid), completed a phase 1-2 study that terminated early due to kidney toxicity when the dose was escalated.

US 2012/0184586 A1 discloses desferrithiocin polyether analogues and uses thereof, for example in treating conditions such as metal overload, oxidative stress, and neoplastic and pre-neoplastic conditions.

Thus, there is a need for improved chelator therapy of metal-mediated conditions.

### 3. SUMMARY

The disclosure relates to methods of treating metal-mediated conditions, for example in a subject having one of the metal-mediated conditions described herein. The disclosure further relates to reducing the toxicity associated with metal chelators used to treat metal-mediated conditions. In one aspect, the disclosure relates to reducing the toxicity associated with SP-420 ((S)-4,5-dihydro-2-[2-hydroxy-4- (3,6-dioxaheptyloxy)phenyl]-4-methyl-4-thiazolecarboxylic acid) and pharmaceutically acceptable salts, solvates, and hydrates thereof (collectively, "SP-420 compounds") in treatment of iron-mediated conditions. Any references in the description to methods of treatment refer to the compound of the present invention for use in a method for treatment of the human (or animal) body by therapy.

There appears to be a strong link between effective chelation and toxicity. HBED was not bioavailable and SPD602 was not effective and neither drug displayed overt toxicity. This is not surprising given that for most small molecule drugs, as dose is escalated, there is increasing exposure, efficacy, and side effects. The side effects can be mechanistically related to the drug's mode of action, off-target effects on other receptors or pathways, or idiosyncratic effects such as allergic reactions. In the case of iron chelators, side effects appear to be mechanistic. Iron chelators remove iron from cells which is the intended effect since iron overload is injurious to cells, particularly in the heart, liver, and pancreas. However, iron chelators also remove iron from cells in other organs such as the kidney and intestine, which frequently are the target organs of toxicity. Cells in these organs can become dysfunctional since these cells require available (non-chelated) intracellular iron for normal cellular processes such as respiration. Cells that are most likely to be affected are those that are most metabolically active since iron containing proteins are abundant in the mitochondria and cell function and survival are dependent on an abundant supply of energy. This may explain why side-effects can be severe or fatal with iron chelators, especially at high doses or when iron stores are depleted. As a class, iron chelators have a low therapeutic index but, in the absence of alternatives, chelators are widely prescribed for transfusion-related iron overload.

The disclosure is based on the insight that it is possible to to dissociate efficacy from side-effects by modifying the dose or dosage regimen of chelators, such as approved iron chelators and SP-420 compounds. Without being bound by theory, the inventors believe that the frequency of dosing can be modified so as to minimize metal (e.g., iron) removal from sensitive tissues without compromising the goal of removing metal from the body overall or from the intended target organs. Specifically, by reducing the duration or frequency of exposure without necessarily or significantly decreasing the total chelator dose or total body metal clearance, it is expected that cells in sensitive organs (*e.g.,* kidney, intestine) are protected from toxic side effects because the reduced exposure allows the cells to recover from the effects of chelation. Providing an "off" chelator period (or cellular recovery period) would allow these cells to preserve critical metabolic functions such as respiration and energy production. Because the dosing regimens described herein are based on the premise that reducing the duration and/or frequency of metal chelator administration allows cells in sensitive organ to recover, the regimens are referred to as "cellular recovery dosing regimens" or "cellular recovery regimens" in short.

The invention is defined in the claims.

The present invention provides a compound for use in a method of treating iron overload in a subject, the method comprising orally administering to the subject the compound (i) every other day, (ii) three or four times per week but no more frequently than every other day; or (iii) every other day with a weekend break, preferably on Monday, Wednesday and Friday with a weekend break, wherein the compound is (S)-4,5-dihydro-2-[2-hydroxy-4-(3,6-dioxaheptyloxy)phenyl]-4-methyl-4-thiazolecarboxylic acid or a pharmaceutically acceptable salt, solvate, or hydrate thereof.

In some embodiments, the compound is administered every other day.

In some embodiments, the compound is administered three or four times per week but no more frequently than every other day.

In some embodiments, the compound is administered every other day with a weekend break, preferably on Monday, Wednesday and Friday with a weekend break.

In some embodiments, the compound is administered to the subject four times per week. In other embodiments, the compound is administered three times per week.

The compound may be administered 15-25 times per month. The compound may be administered 12-18 times per month. The compound may be administered 15-20 times per month.

The compound may be administered every 28 to 72 hours. In some embodiments, the compound is administered every 42 hours. In some embodiments, the compound is administered every 48 hours. In some embodiments, the compound is administered every two days. In some embodiments, the compound is administered every 60 hours. In some embodiments, the compound is administered every 72 hours. In some embodiments, the compound is administered every three days.

The compound may be administered every 32 to 48 hours. The compound may be administered every 36 to 48 hours. The compound may be administered every 32 to 60 hours. The compound may be administered every 36 to 60 hours.

In some embodiments, the compound is administered every 48 to 60 hours.

The compound may be administered every 32 to 72 hours. The compound may be administered every 36 to 72 hours.

In some embodiments, the compound is administered every 48 to 72 hours.

In some embodiments, the compound is administered for a period of at least one week, at least one month, at least three months, at least six months, at least one year, or is administered indefinitely.

In some embodiments, a dose that is greater than the standard daily dose is given at each administration.

In some embodiments, the aggregate weekly dose administered is equal to the standard aggregate weekly dose. In other embodiments, the aggregate weekly dose administered is less than the standard aggregate weekly dose. In some embodiments, the aggregate weekly dose administered is 0.75 to 0.9 times the standard aggregate weekly dose.

In some embodiments, the standard daily dose is given at each administration and/or the aggregate weekly dose administered is equal to the standard aggregate weekly dose. In other embodiments, the aggregate weekly dose administered is greater than the standard aggregate weekly dose. In some embodiments, the aggregate weekly dose administered is 1.25 to 2 times the standard aggregate weekly dose.

In some embodiments, the compound is administered at a dose of 18-100 mg/kg.

In some embodiments, the compound is administered at a dose of 18-30 mg/kg. For example, in some embodiments, the compound is administered at a dose of 24 mg/kg.

In some embodiments, the compound is administered at a dose of 40-60 mg/kg.

In some embodiments, the compound is administered at a dose of 60-84 mg/kg. For example, in some embodiments, the compound is administered at a dose of 72 mg/kg.

In some embodiments, the compound is administered at a dose ranging between 500 mg and 10 g. In certain embodiments, the compound is administered at a dose ranging between 1 g and 5 g.

In some embodiments, the compound is administered at a total weekly dose of 54-400 mg/kg.

In some embodiments, the compound is administered at a total weekly dose of 54-100 mg/kg.

In some embodiments, the compound is administered at a total weekly dose of 100-200 mg/kg.

In some embodiments, the compound is administered at a total weekly dose of 200-300 mg/kg.

In some embodiments, the compound is administered at a total weekly dose of 300-400 mg/kg.

In some embodiments, the compound is administered at a total weekly dose ranging between 1.5 g and 30 g. In some embodiments, the compound is administered at a total weekly dose ranging between 3 g and 30 g. In some embodiments, the compound is administered at a total weekly dose ranging between 5 g and 20 g.

The compound is an iron chelator. In some embodiments, the compound is (S)-4,5-dihydro-2-[2-hydroxy-4-(3,6-dioxaheptyloxy)phenyl]-4-methyl-4-thiazolecarboxylic acid or a pharmaceutically acceptable salt thereof.

In some embodiments, the compound is SP-420 ((S)-4,5-dihydro-2-[2-hydroxy-4-(3,6-dioxaheptyloxy)phenyl]-4-methyl-4-thiazolecarboxylic acid).

In some embodiments, the compound is a pharmaceutically acceptable salt of SP-420. In other embodiments, the compound is a pharmaceutically acceptable solvate of SP-420. In other embodiments, the compound is a pharmaceutically acceptable hydrate of SP-420.

In some embodiments, the iron overload is transfusional iron overload.

In some embodiments, the subject has β-thalassemia. In some embodiments, the subject has β-thalassemia major. In some embodiments, the subject has β-thalassemia intermedia.

In some embodiments, the subject has sickle-cell anemia, Diamond-Blackfan anemia, sideroblastic anemia, chronic hemolytic anemia, off-therapy leukemia, bone marrow transplant, or myelodysplastic syndrome.

In some embodiments, the subject has a hereditary condition resulting in the excess absorption of dietary iron. For example, in some embodiments, the subject has hereditary hemochromatosis or porphyria cutanea tarda.

In some embodiments, the patient has an acquired disease that results in excess dietary iron absorption. For example, in some embodiments, the metal-mediated condition is a liver disease. In some embodiments, the liver disease is hepatitis.

In some embodiments, the iron overload is the result of mal-distribution or redistribution of iron in the body.

In some embodiments, the subject has atransferrinemia, aceruloplasminemia, or Fredreich's ataxia.

### 4. BRIEF DESCRIPTION OF THE FIGURES

**FIG. 1A-1B** show representative KIM-1 data for rats in Example 2 receiving SP-420 every day (Fig. 1A) or every other day (Fig. 1B).
**FIG. 2** shows KIM-1 strips for the rats in Example 2 after having been given a cumulative dose of SP-420 of 2600 mg/kg.
**FIG. 3** shows KIM-1 strips for the rats in Example 2 that had been administered 162.5 mg/kg SP-420 daily for 16 days at the end of the dosing period (left side) and five days post administration (right side).
**FIG. 4** shows a 24-hour urine sample of a rat administered SP-420 daily at a dose of 200 mg/kg/day.
**FIG. 5** shows average KIM-1 data for the rats of Example 2.

### 5. DETAILED DESCRIPTION

### 5.1 Metal-Mediated Conditions

Metal ions are critical to the proper functioning of living systems. Ions such as Fe³⁺, Zn²⁺, Cu²⁺, Ca²⁺, and Co³⁺, to name but a few, can be found in the active sites of over a third of known enzymes and other functional proteins such as RNA polymerase, DNA transcription factors, cytochromes P450s, hemoglobin, myoglobin, and coenzymes such as vitamin B₁₂. There, these metals serve to facilitate oxidation and reduction reactions, stabilize or shield charge distributions, and orient substrates for reactions.

However, the body has a limited ability to absorb and excrete metals, and an excess can lead to toxicity. The principles underlying the present disclosure can be applied to any condition in which an excess of metals is the causative agent of a health condition.

As one example, an excess of iron, whether derived from red blood cells chronically transfused, necessary in such conditions such as β-thalassemia major, or from increased absorption of dietary iron such as hereditary hemochromatosis caused by mutation in genes such as HFE can be toxic through the generation by iron of reactive oxygen species such HO·and HOO·. In the presence of Fe²⁺, H₂O₂ is reduced to the hydroxyl radical (HO·), a very reactive species, a process known as the Fenton reaction. The hydroxyl radical reacts very quickly with a variety of cellular constituents and can initiate free radicals and radical-mediated chain processes that damage DNA and membranes, as well as produce carcinogens. The clinical result is that without effective treatment, total body iron progressively increases with deposition in the liver, heart, pancreas, and elsewhere. Iron accumulation may also produce (i) liver disease that may progress to cirrhosis, (ii) diabetes related both to iron-induced decreases in pancreatic β-cell secretion and increases in hepatic insulin resistance and (iii) heart disease, still the leading cause of death in β-thalassemia major and other anemias associated with transfusional iron overload.

As another example, relative excess iron has been associated with increased risk of cardiovascular diseases (*e.g.,* heart disease). There is a strong correlation between serum ferritin levels, inflammatory biomarkers such as C-reactive protein and interleukin-1, and mortality is a subset of patients with peripheral arterial disease; phlebotomy and iron chelation has been used to mitigate that risk. Treatment with an iron chelator is thought to reduce iron stores, reduce serum ferritin and potentially reduce the incidence of heart disease and stroke.

As another example, ions with little or no endogenous function may find their way into the body and effect damage. Heavy metal ions such as Hg²⁺ can replace ions such as Zn²⁺ in metalloproteins and render them inactive, resulting in serious acute or chronic toxicity that can end in a patient's death or in birth defects in that patient's children. Even more significantly, radioactive isotopes of the lanthanide and actinide series can visit grave illness on an individual exposed to them by mouth, air, or skin contact. Such exposure could result not only from the detonation of a nuclear bomb or a "dirty bomb" composed of nuclear waste, but also from the destruction of a nuclear power facility.

Any of the foregoing iron overload conditions, as well as those identified in Section 3 ("Summary"), can benefit from the cellular recovery dosing regimens according to the claimed invention. The present disclosure encompasses metal chelators, including metal chelators currently approved for therapy, e.g, deferasirox, for use in methods of treating such metal mediated conditions using the regimens described in Section 5.3 or identified using the assays identified in Section 5.4 while the claimed invention relates to (S)-4,5-dihydro-2-[2-hydroxy-4-(3,6-dioxaheptyloxy)phenyl]-4-methyl-4-thiazolecarboxylic acid or a pharmaceutically acceptable salt, solvate, or hydrate thereof ("the compound") for use in a method of treating iron overload in a subject, the method comprising orally administering to the subject the compound (i) every other day; (ii) three or four times per week but no more frequently than every other day; or (iii) every other day with a weekend break, preferably on Monday, Wednesday and Friday with a weekend break.

### 5.2 Metal Chelators

The cellular recovery dosing regimens of the invention pertain to metal chelating agents which are SP-420 compounds. As used herein, "SP-420 compounds" means SP-420 ((S)-4,5-dihydro-2-[2-hydroxy-4-(3,6-dioxaheptyloxy)phenyl]-4-methyl-4-thiazolecarboxylic acid) and pharmaceutically acceptable salts, solvates, and hydrates thereof.

Various agents for the chelation and decorporation of metal ions in living organisms are in clinical use and others have been disclosed and either are untested clinical or failed to advance in clinical trials due to toxicity. The claimed invention relates to SP-420 compounds. The metal chelator based treatment according to the claimed invention can benefit from reduced toxicity resulting from the cellular recovery regimens described in Section 5.3 or identified using the assays identified in Section 5.4.

Some metal chelators of the disclosure can be administered in the form of pharmaceutically acceptable salts. Pharmaceutically acceptable salts include alkali and alkaline earth metal (e.g., sodium, potassium, magnesium, calcium) salts. In addition, pharmaceutically acceptable salts include amine salts. Preferably, the pharmaceutically acceptable salts are metal-free (e.g., iron-free) salts. As used herein, "metal-free salt" does not encompass alkali and alkali earth metal salts.

### 5.3 Dosing Regimens

The cellular recovery dosing regimens of the disclosure are believed to have a net effect of increasing the therapeutic index of metal chelator therapy by preserving efficacy while minimizing toxicity.

The regimens generally reduce the dose frequency by increasing the period of time between doses (the dosing interval) to allow a period of time where chelator is not present in the cells of the body at sufficiently high concentrations to effectively chelate metal ions. This allows the cells to have functional metal containing proteins in sufficient amounts to have normal cellular function. However, the chelator dose and frequency are sufficient to create a state of metal balance or negative metal balance depending on the therapeutic goal of chelation therapy.

For context, in the case of the chelators currently approved for therapy, in some embodiments of the disclosure the interval between doses is increased by approximately 30% to 300% (increasing the 24 hour interval for once per day dosing by approximately 4 hours to 72 hours). For example, a chelator such as deferasirox is typically given once per day on an empty stomach before the morning meal or with a light, low-fat breakfast. Thus the interval between doses is approximately 24 hours. In one scenario, the second dose would occur at the next day mid-day meal, extending the interval to approximately 28 hours. The third dose would be at the evening meal of the following day and so forth. In another scenario, the second dose would occur at the next day's evening meal, extending the interval to approximately 32 hours. In a third scenario, the second dose would occur 2 days later at the morning meal, extending the interval to approximately 48 hours. The predicted benefit of extending the interval between doses would be to allow the cells adversely affected by chelation of iron the necessary time to recover during an effectively chelator-free period. Although some chelator may be present, it would be at a sufficiently low concentration so as not to interfere with cellular function. In the case of deferasirox (a chelator currently approved for therapy), this is predicted to result in substantially less kidney and gastrointestinal injury. More generally, it is predicted that dose-related toxicity observed with any chelator would be reduced or eliminated with the modified dosing regimen.

In certain aspects the dose administered with the new regimen would be increased to offset the reduced dosing frequency. The dose of chelator given at each administration would therefore be increased such that the intended amount of metal clearance was maintained with the new regimen. The dose can be increased by the addition of 10% to 300% of the original dose. By way of illustration, a 20 mg/kg dose of deferasirox (a chelator currently approved for therapy) is increased to a range of 22 to 60 mg/kg at each administration. All ranges described in the disclosure are inclusive (*e.g.,* a range of 22 to 60 mg/kg encompasses a 22 mg/kg dose and a 60 mg/kg dose).

In some embodiments, the total weekly or monthly dose (the sum of all doses administered in a week or month) is maintained. For instance, if deferasirox (a chelator currently approved for therapy, but not part of the invention) was initially dosed at 20 mg/kg daily and the dosing interval was changed to every other day dosing, then the dose administered would increase to 40 mg/kg administered every other day.

In the dosing regimens described herein, the phrase "weekly doses" when preceded by an integer refers to the number of doses administered in a week. For example, a dosing regimen comprising "fewer than 5 weekly doses" encompasses dosing regimens in which the subject is administered a dose of a metal chelator fewer than five times in a week (*e.g.,* 1, 2, 3, or 4 times in a week). Similarly, the phrase "daily doses" when preceded by an integer refers to the number of doses administered in a day. For example, a dosing regimen comprising "one or two daily doses" encompasses dosing regimens in which the subject is administered a dose of a metal chelator once per day or twice per day. When a specific dose of a metal chelator is provided, unless required otherwise by context, the dose refers to the amount of metal chelator administered in a single dose. The foregoing weekly, daily and specific doses can be administered for a period of at least one week, at least one month, at least three months, at least six months, at least one year, or indefinitely (*e.g.,* for the remainder of a subject's life). Unless required otherwise by context, a period of administration refers to the amount of time between the first and last doses of a metal chelator, rather than the amount of time of a single administration.

The dosing regimen can be modified further to enhance convenience, compliance, and adherence. For instance, every other day dosing can be changed to dosing on three days per week, with, for example a weekend break. In geographies where the weekend is Saturday and Sunday, such a dosing schedule can consist of administering the chelator on Mondays, Wednesdays, and Fridays. It is believed that this would enhance the ability of a patient to remember to take the dose and thereby comply with the recommended regimen. Also, as shown in Example 2, a short break from dosing is expected to minimize chelator toxicity.

The metal chelating efficiency (the percentage of drug administered that is excreted from the body chelated with metal) can be determined with metal balance studies in animals, human clinical trial subjects, or patients or by measuring tissue metal content, for instance in animals using atomic absorption spectroscopy of tissue or in humans by MRI.

Since side-effects from chelators are either predictable or can be anticipated based on screening tests, the between dose interval can be adjusted over time while monitoring side-effects such as abdominal pain or examining the urine or blood for evidence of kidney injury or dysfunction.

In particular embodiments, the metal chelator of the invention is administered according to any of the regimens identified in the claims or in Section 3 ("Summary").

The chelators used in the cellular recovery regimens of the disclosure are preferably in immediate release oral formulations but may also be delayed release oral dosage forms or in parenteral preparations. The cellular recovery regimens of the disclosure can be used for dosing humans (including both adults and children) and non-human animals.

### 5.4 Assays for Optimizing Dosing Regimens

Various cell and animal models can be used to evaluate the optimal dosing intervals for metal chelators. These models probe the toxic effects on metal chelators on cells in sensitive organs to determine dose toxicity and cellular recovery time between doses.

The cell types studied in cell culture can be selected to reflect the cell type adversely affected in humans such as hepatocytes or granulocyte progenitor cells.

To evaluate the effects of metal chelators on renal toxicity, primary proximal tubule kidney cells or immortalized proximal tubule cell lines can be grown in tissue culture (*in vitro*) using defined culture media. Assays exist to quantify the metabolic status (*e.g.,* MTTP assay), proliferation (*e.g.,* cell counts), and vitality (*e.g.,* trypan blue exclusion) of these cells. Metal (*e.g.,* iron) chelators can be added to the culture medium for defined periods of time to model *in vivo* exposure of the kidney. For instance, following daily oral dosing of deferasirox at steady state in human patients, the peak serum concentration is achieved at approximately 2 hours after the dose and the mean elimination half-life of is approximately 12 hours. Deferasirox is highly bound to albumin so albumin would be added to the culture medium. To model this, the cells in culture can be exposed to the high peak serum concentration in the culture medium for 2 hours. The culture medium can be changed with fresh medium containing a lower concentration of deferasirox every 2 hours such that at the end of 14 hours (2 hours of initial high concentration plus 12 hours of declining concentration) the final concentration is approximately half that of the initial concentration. This every 2 hour reduction in deferasirox concentration is continued until the end of the 24 hour period resulting in a concentration that approximately equaled the trough concentration observed in patients taking deferasirox once per day chronically. This dosing scheme can be repeated for a number of days, for instance 4 days. To confirm that toxicity can be reduced using a cellular recovery regimen that incorporates a chelator free period in which the cells are able to recover metabolic functions, the cellular toxicity observed using an exposure schedule based on standard dosing of a metal chelator is then compared to the toxicity observed using an exposure schedule based on a cellular recovery dosing schedule. For example in the case of deferasirox, the cellular assay described above can be carried out using a reduced dosing schedule, for example involving exposing the cells to twice the concentrations of defersirox every other day for the same number of days to achieve the same overall dose.

In the case of other chelators, the assays are designed to compare a typical pharmacokinetic profile of that chelator with a modified profile that incorporates chelator free periods for cellular recovery. Deferiprone, for instance, is administered 3 times per day, typically once in the morning upon arising, once at mid-day, and once in the evening. Thus a typical regimen would result in doses separated by approximately 5 hours with a peak serum concentration achieved after 1 hour and a half-life of 2 hours. This can be mimicked by a high initial deferiprone concentration *in vitro* for 1 hour followed by a reduction in concentration of 50% for 2 hours and another reduction of 50% for a subsequent 2 hours. This cycle is repeated 2 more time to mimic the 3 times per day dosing in patients. To confirm that toxicity can be reduced using a cellular recovery regimen that incorporates a chelator free period in which the cells are able to recover metabolic functions, the cellular toxicity observed using an exposure schedule based on standard dosing of a metal chelator is then compared to a cellular recovery dosing schedule. In the case of deferiprone, the toxicity using an exposure schedule based on the standard dosing schedule is compared to an exposure schedule that results in the same overall dose administered in 2 divided doses at higher concentrations separated by 10 hours or as a single dose separated by 24 hours.

Kidney proximal tubule cells and other cells such as hepatocytes can also be cultured in microfluidic kidney models ("kidney-on-a-chip") or liver models ("liver-on-a-chip") and exposed to chelators in a manner that mimics the pharmacokinetic of various dosing regimens. In one embodiment, the "kidney-on-a-chip" model used to evaluate metal chelator toxicity and cellular recovery periods is that described by Kim and Takayama (Kim et al., 2015, Biofabrication. Volume 8, Number 1 015021, dx.doi.org/10.1088/1758-5090/8/1/015021). As with the simpler cell culture models, the metabolic status and vitality can be assessed in response to cellular recovery dosing regimens *(*e.g., every other day dosing of deferasirox or twice a day or once a day dosing for deferiprone) as compared to standard dosing regimens (*e.g.,* daily dosing of deferasirox or thrice daily dosing for deferiprone).

The frequency of the dosing regimen can also be studied in animals. For instance, the same total dose of chelator (*e.g.,* 30 mg/kg/day) can be given to one group of animals as 10 mg/kg three times per day, while another group of animals can be given 15 mg/kg twice per day, and a third group of animals can be given 30 mg/kg once per day. In an extension of the assay, another groups of animals can be given 60 mg/kg once every other day and still another group of animals can be given 90 mg/kg once every third day. These animals are monitored for overt toxicity and evidence of organ dysfunction (*e.g.,* rise in serum creatinine or abnormal urinalysis or elevation of liver enzymes), or changes in tissues (*e.g.,* vacuolization of kidney proximal tubule cells or accumulation of fat in hepatocytes).

Information obtained from these assays can be used to guide dose selection for human clinical trials to compare standard chelator dosing regimens with modified regimens that incorporate periods of cellular recovery from chelator therapy. The modified regimens can also be studied alone and compared to historical control data, e.g., from trials with standard regimens.

### 6. EXAMPLES

### 6.1 Example 1: Comparison of daily versus every other day dosing of SP-420

This study was performed to compare the toxicity of daily versus every other day dosing of SP-420 (administered as the sodium salt) by assessing levels of kidney injury molecule-1 (KIM-1) and the presence of glucose in the urine (glucosuria). KIM-1 is a sensitive and specific urinary biomarker of kidney injury in both rodent models and humans (see, *e.g.,* Sabbisetti et al., 2014, J Am Soc Nephrol. 25(10):2177-2186). KIM-1 is minimally expressed in normal rat kidneys, but is markedly expressed upon kidney injury (*see, e.g,* Vaidya et al., 2010, Nat Biotechnol. 28(5): 478-485). Glucose filtered by the kidney glomerulus is normally completely reabsorbed by kidney proximal tubule cells and therefore it is very abnormal to detect glucose in the urine. Glucosuria is a conditon where excessively large amounts of glucose are filtered the glomerulus, overwhelming the absorptive mechanism. Glucosuria is generally only detected in diabetes mellitus secondary to hyperglycemia or when the kidney proximal tubule cells become dysfunctional, as in the renal Fanconi syndrome. The renal Fanconi syndrome has been reported to be caused by iron chelator treatment. Resorption of glucose and other filtered substances (e.g., amino acids, phosphorus, uric acid) is highly energy dependant and chelation of kidney proximal tubule cell iron results in decreased mitochondrial energy production leading to cellular dysfunction with decreased resorptive capability.

### 6.1.1. Methods

Male Sprague-Dawley rats (n=5 per group) were given SP-420 orally by gavage once daily (SID) at a dose of 162.5 mg/kg or every other day (EOD) at a dose of 325 mg/kg. Each group of rats received a cumulative dose of 2600 mg/kg of SP-420. Urine was collected from metabolic cages EOD during the course of administration, 5 or 6 days after the last dose, and again 11 or 12 days after the last dose, and assessed for its KIM-1 content and subjected to a 10-parameter urine dipstick analysis. The urine from the animals administered SP-420 EOD was collected during the 24 hours immediately following the dose of the drug.

### 6.1.2. Results

All of the SID and EOD-treated rats survived the exposure to the drug. However, one rat in the 162.5 mg/kg SID group was euthanized 3 days post-drug due to ongoing weight loss and deteriorating condition. Urinary KIM-1 levels increased in both groups, but the increase was not as significant in animals given the drug EOD. Changes in the KIM-1 levels largely returned to baseline levels by 5 or 6 days post-drug. Glucosuria was observed in 4/5 of the rats administered SP-420 SID, but in none of the rats that were administered SP-420 EOD.

This study shows that administering SP-420 every other day rather than every day reduces toxicity.

### 6.2 Example 2: Repeat study of Example 1

In Example 1, "recovery" KIM-1 levels were not measured until 5 or 6 days post last dose. Although it was clear that the KIM-1 levels dropped dramatically during this time period, the study in Example 1 was not designed to show how quickly the parameters returned to baseline levels. In addition, in Example 1 urine was collected from the rats given the chelator EOD in the 24 hours immediately following the dose of the drug but no data were collected for urine produced in the 24-48 hour time period leading up to the next dose of SP-420. In order to determine what occurred during this "off" day and to determine whether urinary KIM-1 levels decrease between doses, the study of Example 1 was repeated with slight modifications.

### 6.2.1. Methods

Ten male Sprague-Dawley rats were randomly divided into two groups of five and dosed as in Example 1. Unlike in Example 1, in which the rats were housed in the metabolic cages EOD, the animals in this repeat study were housed individually in metabolic cages throughout the course of the dosing regimen, and for 6 days thereafter to allow for the collection of urine during a recovery period. Urine was collected at 24-h intervals throughout the course of the study and assessed for its KIM-1 content. For the rats receiving the drug EOD, KIM-1 data was obtained both 0-24 hours immediately following the dose of the drug, as well as from the "off" day (24-48 hours post drug). The rats were weighed once daily. Changes in body weight were calculated from the animals' baseline weight versus their weight on the day that the last dose of the drug was given.

### 6.2.2. Results

All of the rats survived the exposure to the test drug. One rat from the 162.5 mg/kg SID group was euthanized 4 days post-last dose due to weight loss and overall deteriorating condition.

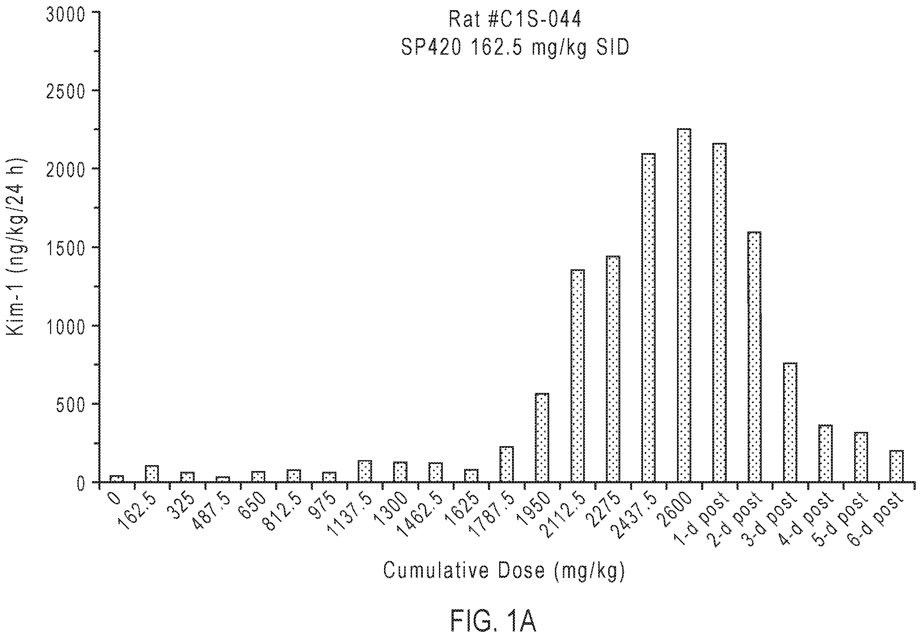

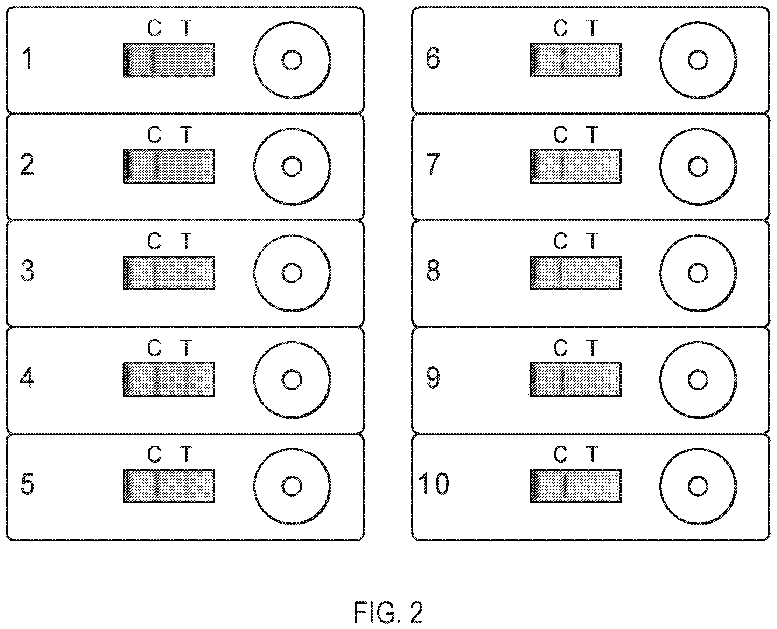

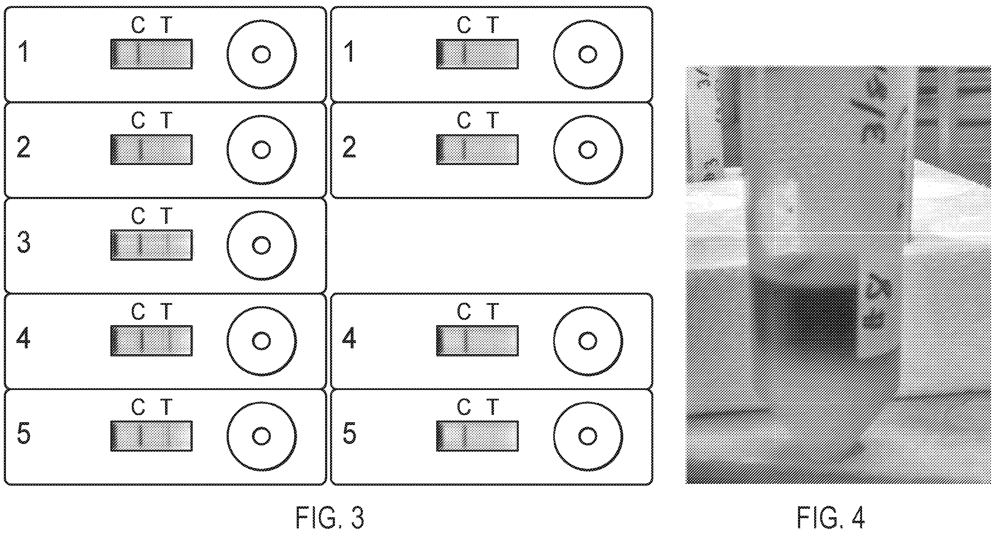

Representative KIM-1 data for a rat in each group is shown in Fig. 1A-1B. Although Fig. 1A-1B show that SP-420 induced increased KIM-1 levels in both groups of animals, the values returned to near baseline levels very quickly. The increase in KIM-1 levels in rats administered SP-420 EOD was not as significant in animals administered SP-420 SID. This is depicted in the KIM-1 strips shown in Fig. 2. Strips numbered 1-5 were from rats administered SP-420 SID at 162.5 mg/kg/day, while strips numbered 6-10 were from rats administered SP-420 at 325 mg/kg EOD. The "C" line is an internal standard, while the "T" line represents the results for the test animal. The "T" line in strips 3-5 are quite pronounced, while very little KIM-1 was observed in any of strips 6-10 except strip 7. KIM-1 levels decreased from the last administration to 5-days post-administration, as shown in Fig. 3. Fig. 3 shows KIM-1 strips from the rats administered SP-420 SID at the end of the administration period (left side) and 5 days post-administration (right side). Very little KIM-1 was observed in strip #4 or #5 five days post-drug. The rat corresponding to strip #3 was euthanized prior to obtaining the 5 days post-administration sample.

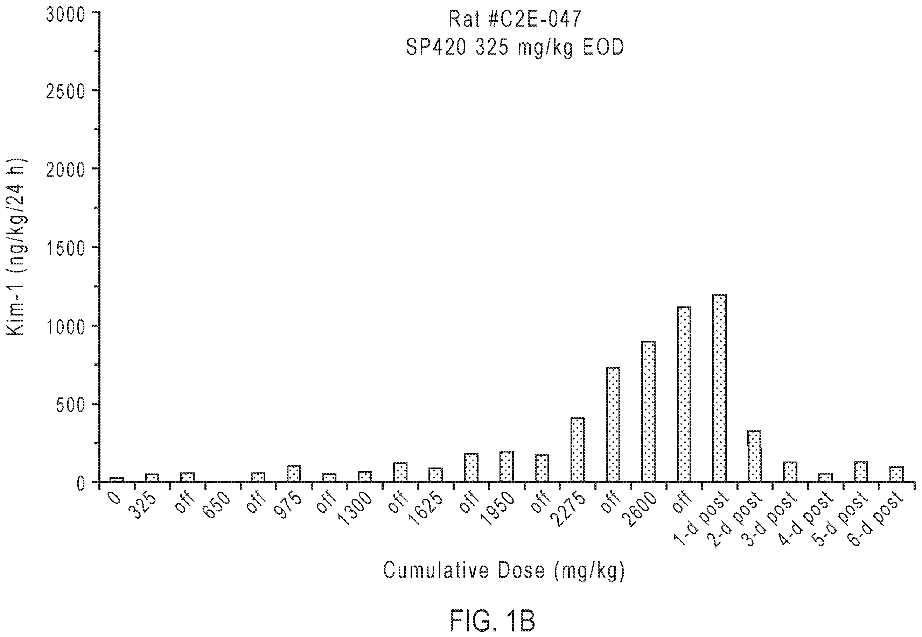

Under the conditions of the study, KIM-1 levels did not decrease in rats treated EOD on the "off" day (as shown in representative Fig. 1B). This was also observed in the recovery data of the rats treated with SP-420 SID, where the KIM-1 levels were virtually unchanged between what was seen on the last day of dosing versus the quantity of KIM-1 in the urine 24 hours post drug. However, the lack of a decrease in KIM-1 between doses in the EOD animals, or within 24 hours of stopping the drug in the SID rodents, is not surprising. The rats in these studies generally excrete less than 10-15 mL of urine per day. Due to such a small quantity of urine being produced, it is quite likely that there was significant carry over of KIM-1, glucose, etc., from one day to the next. For example, the picture shown in Fig. 4 depicts the 24-hour urine sample of a rat treated SID with SP-420 at a dose of 200 mg/kg/day. The volume of urine produced on that day was only 14.6 mL. While the bottom half of the tube contained normal looking urine, the top half contained a layer of frank blood. Based on the "layering effect" of normal urine versus urine containing blood observed during this single 24-hour sample, it is not unreasonable to expect that some of the KIM-1 or glucose present at the end of one 24-hour collection period would continue to be excreted into the following day's urine specimen as well.

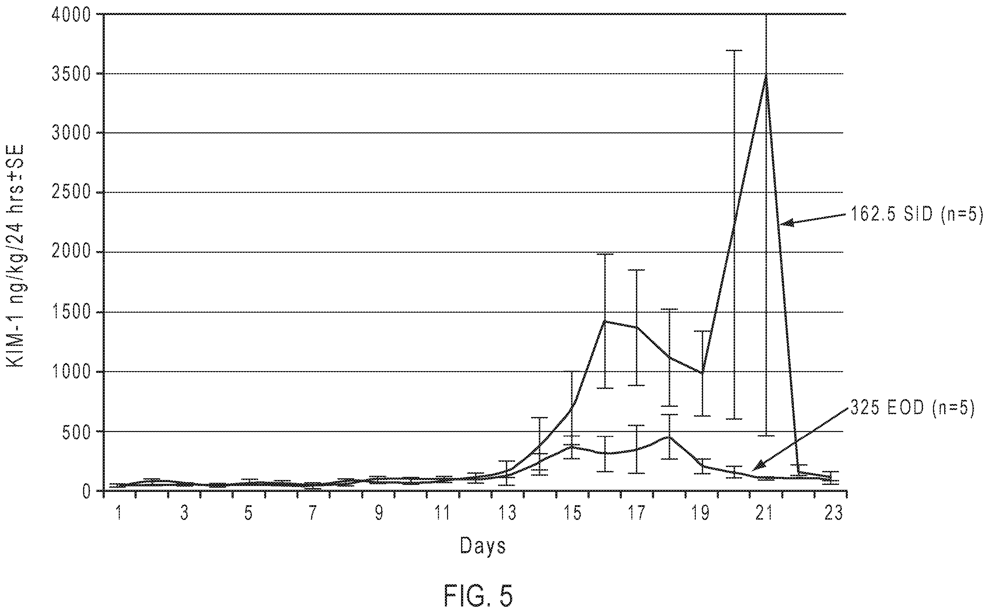

Average KIM-1 data for all rats in each group is shown in Fig. 5. Although Fig. 5 shows that SP-420 induced increased average KIM-1 levels in both groups of animals, the average values returned to near baseline levels very quickly. The increase in average KIM-1 levels in rats administered SP-420 EOD was not as significant in animals administered SP-420 SID.

Glucosuria was observed in 3/5 rats given SP-420 SID. During the recovery period, glucosouria continued to be observed in all three of the rats one day post-drug, in 1/3 of the rats 2 days post-drug, and in none of the affected rats by 3 days post-last dose. Glucosuria was not observed in any of the rats administered SP-420 EOD at 325 mg/kg.

### 6.2.3. Summary

Example 2 shows that administering SP-420 every other day versus once daily reduces toxicity. This is reflected both in terms of urinary KIM-1 excretion, as well as the presence/absence of glucose in the test animals' urine. The rapid decrease in urinary KIM-1 levels on days 2-6 post drug provides strong support for the idea of administering the chelator to patients on a three days a week dosing schedule with a weekend break, e.g., a Monday, Wednesday, Friday dosing schedule. With a Monday, Wednesday and Friday dosing schedule the weekend break would, if necessary, allow the kidneys time to recover before dosing resumed on Monday.

### 6.3. Example 3: Comparison of daily versus every other day dosing of SP-420 at different doses

Studies similar to those described in Examples 1 and 2 were performed with 250 mg/kg EOD, 250 mg/kg SID, 500 mg/kg EOD, 200 mg/kg SID and 400 mg/kg EOD of SP-420. The results of the studies, together with the results of the studies described in Examples 1 and 2, are summarized in Table 1, below. As shown in Table 1, glucosuria and KIM-1 levels were consistenly lower for EOD dosing regimens compared to SID regimens for the same cumulative dose. Taken together, the data support for the idea of administering SP-420 to patients on an every other day or a three times a week (e.g., Monday, Wednesday, Friday) dosing schedule.

| Dose & Frequency: | Control | 250 mg/kg EOD | 250 mg/kg SID | 500 mg/kg EOD | 162.5 mg/kg SID | 325 mg/kg EOD | 200 mg/kg SID | 400 mg/kg EOD |
|---|---|---|---|---|---|---|---|---|
| Number of Rats per Group | 5 | 5 | 5 | 5 | 10 | 10 | 5 | 5 |
| Maximum Cumulative Dose (mg/kg) | 0 | 2500 | 2500 | 2500 | 2600 | 2600 | 2400 | 2400 |
| Survival | 5/5 | 5/5 | 2/5 | 0/5 | 8/10 | 10/10 | 4/5 | 3/5 |
| Deaths @ | All | All | 2 @ 2000 | 1 @ 1500 | 2 @ 2600 | All | 1 @ 2000 | 1 @ 2000 |
| | | | | 2 @ 2000 | 1-3d post | | | |
| Cumulative Dose (mg/kg) | Survived | Survived | 1 @ 2500 | | | Survived | | 1 @ 2400 |

| | | | | 2 @ 2500 | 1-4d post | | | |
|---|---|---|---|---|---|---|---|---|
| Weight Change (%) | +16.0 ± 1.4 | +8.5 ± 3.9 | -5.2 ± 4.0 | -5.2 ± 5.0 | -1.1 ± 5.0 | +0.8 ± 2.5 | -4.9 ± 5.3 | -2.8 ± 5.6 |
| Weight Change (Range, %) | +13.7 to +17.0 | +3.1 to +12.4 | -9.1 to +0 | -13.1 to -1.2 | -7.7 to +5.5 | -3.0 to +3.4 | -8.3 to +4.4 | -8.8 to +2.9 |
| Urine Dipstick | | | | | | | | |
| Glucosuria | 0/5 | 0/5 | 4/5 | 1/5 | 7/10 | 0/10 | 3/5 | 1/5 |

| Hematuria (Frank) | 0/5 | 0/5 | 1/5 | 1/5 | 0/10 | 0/10 | 2/5 | 0/5 |
|---|---|---|---|---|---|---|---|---|
| pH ≤ 5 | 0/5 | 0/5 | 3/5 | 2/5 | 5/10 | 0/10 | 4/5 | 2/5 |
| Specific gravity ≥ 1.030 | 0/5 | 0/5 | 2/5 | 1/5 | 4/10 | 2/10 | 3/5 | 1/5 |
| Kidney Injury Molecule (KlM-1) | | | | | | | | |
| KIM-1 ≥ 250 ng/kg/24 h | 0/5 | 0/5 | 5/5 | 4/5 | 8/10 | 9/10 | 5/5 | 4/5 |
| KIM-1 ≥ 500 ng/kg/24 h | 0/5 | 0/5 | 5/5 | 2/5 | 7/10 | 4/10 | 5/5 | 2/5 |
| KIM-1 ≥ 1000 ng/kg/24 h | 0/5 | 0/5 | 3/5 | 1/5 | 7/10 | 3/10 | 5/5 | 2/5 |
| KIM-1 ≥ 2000 ng/kg/24 h | 0/5 | 0/5 | 0/5 | 0/5 | 4/10 | 1/10 | 1/5 | 1/5 |
| **Table 1: Tolerability of SP-420 given Orally by Gavage to Male Sprague-Dawley Rats (Sodium Salt)** | | | | | | | | |

## Claims

1. A compound for use in a method of treating iron overload in a subject, the method comprising orally administering to the subject the compound:
(i) every other day;
(ii) three or four times per week but no more frequently than every other day; or
(iii) every other day with a weekend break, preferably on Monday, Wednesday and Friday with a weekend break,
wherein the compound is (S)-4,5-dihydro-2-[2-hydroxy-4-(3,6-dioxaheptyloxy)phenyl]-4-methyl-4-thiazolecarboxylic acid or a pharmaceutically acceptable salt, solvate, or hydrate thereof.

2. The compound for use as claimed in claim 1, wherein the compound is administered every other day.

3. The compound for use as claimed in claim 1, wherein the compound is administered three or four times per week but no more frequently than every other day.

4. The compound for use as claimed in claim 1, wherein the compound is administered every other day with a weekend break, preferably on Monday, Wednesday and Friday with a weekend break.

5. The compound for use as claimed in any one of claims 1 to 4, wherein the compound is administered for a period of at least one week, at least one month, at least three months, at least six months, at least one year, or is administered indefinitely.

6. The compound for use as claimed in any one of claims 1 to 5, wherein the compound is administered at a dose of 18-100 mg/kg.

7. The compound for use as claimed in claim 6, wherein the compound is administered at a dose of 18-30 mg/kg, 40-60 mg/kg, or 60-84 mg/kg.

8. The compound for use as claimed in any one of claims 1 to 7, wherein the compound is administered at a total weekly dose of 54-400 mg/kg.

9. The compound for use as claimed in claim 8, wherein the compound is administered at a total weekly dose of 54-100 mg/kg, 100-200 mg/kg, 200-300 mg/kg, or 300-400 mg/kg.

10. The compound for use as claimed in any one of claims 1 to 9, wherein the compound is (S)-4,5-dihydro-2-[2-hydroxy-4-(3,6-dioxaheptyloxy)phenyl]-4-methyl-4-thiazolecarboxylic acid or a pharmaceutically acceptable salt thereof.

11. The compound for use as claimed in any one of claims 1 to 10, wherein the subject has β-thalassemia.

12. The compound for use as claimed in claim 11, wherein the subject has β-thalassemia major.

13. The compound for use as claimed in claim 11, wherein the subject has β-thalassemia intermedia.

14. The compound for use as claimed in any one of claims 1 to 10, wherein the subject has sickle-cell anemia, Diamond-Blackfan anemia, sideroblastic anemia, chronic hemolytic anemia, off-therapy leukemia, bone marrow transplant, or myelodysplastic syndrome.

## Patentansprüche

1. Verbindung zur Verwendung in einem Verfahren zur Behandlung der Eisenspeicherkrankheit in einem Individuum, wobei das Verfahren das orale Verabreichen der Verbindung an das Individuum:
(i) alle zwei Tage;
(ii) drei- oder viermal pro Woche, aber nicht häufiger als alle zwei Tage; oder
(iii) alle zwei Tage mit einer Wochenend-Pause, vorzugsweise Montag, Mittwoch und Freitag mit einer Wochenend-Pause,
umfasst,
wobei die Verbindung (S)-4,5-Dihydro-2-[2-hydroxy-4-(3,6-dioxaheptyloxy)phenyl]-4-methyl-4-thiazolcarbonsäure oder ein pharmazeutisch annehmbares Salz, Solvat oder Hydrat davon ist.

2. Verbindung zur Verwendung nach Anspruch 1, wobei die Verbindung alle zwei Tage verabreicht wird.

3. Verbindung zur Verwendung nach Anspruch 1, wobei die Verbindung drei- oder viermal pro Woche, aber nicht öfter als alle zwei Tage verabreicht wird.

4. Verbindung zur Verwendung nach Anspruch 1, wobei die Verbindung alle zwei Tage mit einer Wochenend-Pause, vorzugsweise Montag, Mittwoch und Freitag mit einer Wochenend-Pause verabreicht wird.

5. Verbindung zur Verwendung nach einem der Ansprüche 1 bis 4, wobei die Verbindung über einen Zeitraum von zumindest einer Woche, zumindest einem Monat, zumindest drei Monaten, zumindest sechs Monaten, zumindest einem Jahr oder auf unbestimmte Zeit verabreicht wird.

6. Verbindung zur Verwendung nach einem der Ansprüche 1 bis 5, wobei die Verbindung in einer Dosis von 18 bis 100 mg/kg verabreicht wird.

7. Verbindung zur Verwendung nach Anspruch 6, wobei die Verbindung in einer Dosis von 18 bis 30 mg/kg, 40 bis 60 mg/kg oder 60 bis 84 mg/kg verabreicht wird.

8. Verbindung zur Verwendung nach einem der Ansprüche 1 bis 7, wobei die Verbindung in einer wöchentlichen Gesamtdosis von 54 bis 400 mg/kg verabreicht wird.

9. Verbindung zur Verwendung nach Anspruch 8, wobei die Verbindung in einer wöchentlichen Gesamtdosis von 54 bis 100 mg/kg, 100 bis 200 mg/kg, 200 bis 300 mg/kg oder 300 bis 400 mg/kg verabreicht wird.

10. Verbindung zur Verwendung nach einem der Ansprüche 1 bis 9, wobei die Verbindung (S)-4,5-Dihydro-2-[2-hydroxy-4-(3,6-dioxaheptyloxy)phenyl]-4-methyl-4-thiazol-carbonsäure oder ein pharmazeutisch annehmbares Salz davon ist.

11. Verbindung zur Verwendung nach einem der Ansprüche 1 bis 10, wobei das Individuum an β-Thalassämie leidet.

12. Verbindung zur Verwendung nach Anspruch 11, wobei das Individuum an β-Thalassaemia major leidet.

13. Verbindung zur Verwendung nach Anspruch 11, wobei das Individuum an β-Thalassaemia intermedia leidet.

14. Verbindung zur Verwendung nach einem der Ansprüche 1 bis 10, wobei das Individuum an Sichelzellenanämie, Diamond-Blackfan-Anämie, sideroblastischer Anämie, chromischer hämolytischer Anämie, Leukämie ohne Therapie, einem Knochenmark-Transplantat oder dem myelodysplastischen Syndrom leidet.

## Revendications

1. Composé pour utilisation dans un procédé de traitement d'une surcharge en fer chez un sujet, le procédé comprenant l'administration orale au sujet du composé :
(i) un jour sur deux ;
(ii) trois ou quatre fois par semaine, mais pas plus fréquemment qu'un jour sur deux ; ou
(iii) un jour sur deux avec une pause de week-end, de préférence le lundi, le mercredi et le vendredi avec une pause de week-end,
dans lequel le composé est l'acide (S)-4,5-dihydro-2-[2-hydroxy-4-(3,6-dioxaheptyloxy)phényl]-4-méthyl-4-thiazolecarboxylique ou un sel, un solvate ou un hydrate pharmaceutiquement acceptable de celui-ci.

2. Composé pour utilisation selon la revendication 1, dans lequel le composé est administré un jour sur deux.

3. Composé pour utilisation selon la revendication 1, dans lequel le composé est administré trois ou quatre fois par semaine mais pas plus fréquemment qu'un jour sur deux.

4. Composé pour utilisation selon la revendication 1, dans lequel le composé est administré un jour sur deux avec une pause de week-end, de préférence le lundi, le mercredi et le vendredi avec une pause de week-end.

5. Composé pour utilisation selon l'une quelconque des revendications 1 à 4, dans lequel le composé est administré pendant une période d'au moins une semaine, au moins un mois, au moins trois mois, au moins six mois, au moins un an, ou est administré indéfiniment.

6. Composé pour utilisation selon l'une quelconque des revendications 1 à 5, dans lequel le composé est administré à une dose de 18 à 100 mg/kg.

7. Composé pour utilisation selon la revendication 6, dans lequel le composé est administré à une dose de 18 à 30 mg/kg, de 40 à 60 mg/kg ou de 60 à 84 mg/kg.

8. Composé pour utilisation selon l'une quelconque des revendications 1 à 7, dans lequel le composé est administré à une dose hebdomadaire totale de 54 à 400 mg/kg.

9. Composé pour utilisation selon la revendication 8, dans lequel le composé est administré à une dose hebdomadaire totale de 54 à 100 mg/kg, de 100 à 200 mg/kg, de 200 à 300 mg/kg ou de 300 à 400 mg/kg.

10. Composé pour utilisation selon l'une quelconque des revendications 1 à 9, dans lequel le composé est l'acide (S)-4,5-dihydro-2-[2-hydroxy-4-(3,6-dioxaheptyloxy)phényl]-4-méthyl-4-thiazolecarboxylique ou un sel pharmaceutiquement acceptable de celui-ci.

11. Composé pour utilisation selon l'une quelconque des revendications 1 à 10, dans lequel le sujet est atteint de β-thalassémie.

12. Composé pour utilisation selon la revendication 11, dans lequel le sujet est atteint de β-thalassémie majeure.

13. Composé pour utilisation selon la revendication 11, dans lequel le sujet est atteint de β-thalassémie intermédiaire.

14. Composé pour utilisation selon l'une quelconque des revendications 1 à 10, dans lequel le sujet présente une anémie falciforme, une anémie de Diamond-Blackfan, une anémie sidéroblastique, une anémie hémolytique chronique, une leucémie hors thérapie, une greffe de moelle osseuse ou un syndrome myélodysplasique.
